# EUROPEAN PATENT APPLICATION

(11) **EP 4 696 767 A1**
(43) Date of publication of application: **18.02.2026**
(21) Application number: 25193807.2
(22) Date of filing: 04.08.2025
(51) Int. Cl.: C12M 1/26, C12M 1/32

(54) **CELL SUCTION SYSTEM**

(30) Priority: 16.08.2024 JP 2024136952
(71) Applicant: Yokogawa Electric Corporation, Tokyo 180-8750 (JP)
(72) Inventor: HARAMI, Satoru, Musashino-shi, Tokyo 180-8750 (JP); IMAI, Yuya, Musashino-shi, Tokyo 180-8750 (JP); KUWATA, Masahiro, Musashino-shi, Tokyo 180-8750 (JP)
(74) Representative: Stöckeler, Ferdinand

(57) **Abstract**

A cell suction system that can accurately move a needle tip of a needle portion of a chip to the position of a cell to be sucked, after a chip connection shaft of a suction unit is connected to a chip base of the chip held in a chip container is provided. A cell suction system includes a suction unit and a chip container holder. The suction unit includes a chip connection shaft to be connected to a chip base of a chip held in a chip container, and causes the chip to suck and hold a cell component of a cell or an entire cell from a needle tip of a needle portion, in a state in which the chip is connected to the chip connection shaft. The chip container holder holds the chip container so as to allow movement of the chip and the chip container relative to the chip connection shaft, to eliminate axial misalignment when the chip connection shaft is connected to the chip base of the chip held in the chip container.

## Description

### TECHNICAL FIELD

The present disclosure relates to a cell suction system.

### BACKGROUND

Cell suction systems that cause a chip to suck and hold a cell component of a cell or an entire cell from a needle tip of a needle portion are known. For example, see Patent Literatures (PTLs) 1 to 6.

### CITATION LIST

### Patent Literature

PTL 1: JP 6066110 B2
PTL 2: JP 6090387 B2
PTL 3: JP 6787274 B2
PTL 4: JP 6953888 B2
PTL 5: JP 2023-18912 A
PTL 6: JP 2023-17601 A

### SUMMARY

The present disclosure aims to provide a cell suction system that can accurately move a needle tip of a needle portion of a chip to the position of a cell to be sucked, after a chip connection shaft of a suction unit is connected to a chip base of the chip held in a chip container.

An aspect of the present disclosure is as follows.
[1] A cell suction system including:
   a suction unit including a chip connection shaft to be connected to a chip base of a chip held in a chip container, the suction unit causing the chip to suck and hold a cell component of a cell or an entire cell from a needle tip of a needle portion, in a state in which the chip is connected to the chip connection shaft; and
   a chip container holder that holds the chip container so as to allow movement of the chip and the chip container relative to the chip connection shaft, to eliminate axial misalignment when the chip connection shaft is connected to the chip base of the chip held in the chip container.
[2] The cell suction system according to [1], including the chip container,
   wherein the chip container holder includes:
   a placement portion on which the chip container is placed; and
   a chip container regulator that regulates movement of the chip container in a horizontal direction while allowing sliding of the chip container on the placement portion, to eliminate the axial misalignment when the chip connection shaft is connected to the chip base of the chip held in the chip container.
[3] The cell suction system according to [2], wherein
   the chip container has four corners in a top view, and
   only the four corners of the chip container are placed on the placement portion of the chip container holder.
[4] The cell suction system according to any one of [1] to [3], wherein
   the chip container holder has a placement portion on which the chip container is placed, and
   the placement portion includes:
      a base material; and
      a surface layer applied to an upper surface of the base material, the surface layer having a lower coefficient of friction than the upper surface.
[5] The cell suction system according to any one of [1] to [4], including:
   a first holder that holds the chip container; and
   a second holder that holds the first holder while allowing movement of the first holder relative to the second holder, to eliminate the axial misalignment when the chip connection shaft is connected to the chip base of the chip held in the chip container.
[6] The cell suction system according to [5], wherein the second holder includes:
   a second holder main body; and
   a rolling element rollably held by the second holder main body, the first holder being placed on the rolling element.
[7] The cell suction system according to [6], wherein the rolling element is in the shape of a ball.
[8] The cell suction system according to [6] or [7], wherein three or more rolling elements are provided.
[9] The cell suction system according to any one of [6] to [8], wherein the second holder main body includes:
   a reduced diameter support surface whose diameter gradually reduces downward, the reduced diameter support surface rotatably supporting the rolling element by slidably abutting a lower surface of the rolling element; and
   an ascent regulation portion that regulates upward movement of the first holder relative to the second holder main body, so that the rolling element does not get out of the reduced diameter support surface.
[10] The cell suction system according to any one of [5] to [9], wherein the chip container holder includes a positioning portion that changes a state between a positioning state in which the first holder is positioned relative to the second holder in a horizontal direction and a positioning release state in which positioning is released.
[11] The cell suction system according to any one of [5] to [10], wherein the first holder includes:
   a first holder main body on which the chip container is placed; and
   a chip container positioning portion that positions the chip container on the first holder main body.
[12] The cell suction system according to any one of [1] to [11], including:
   a chip connection operation area in which the chip connection shaft is connected to the chip held in the chip container; and
   a cell suction operation area in which the cell component of the cell or the entire cell is sucked into the chip,
   wherein an ambient temperature of the chip connection operation area is lower than that of the cell suction operation area.
[13] The cell suction system according to any one of [1] to [12], including:
   an imaging device configured to image the cell component of the cell or the entire cell contained in a cell container; and
   a control device configured to control the suction unit based on an imaging result from the imaging device, so that the cell component of the cell or the entire cell is sucked into and held in the chip, in a state in which the chip is connected to the chip connection shaft.
[14] The cell suction system according to any one of [1] to [13], including a conveyance device configured to convey the suction unit from a position in which the chip connection shaft is connected to the chip held in the chip container, to a position in which the cell component of the cell or the entire cell contained in a cell container is sucked into the chip.
[15] The cell suction system according to [14], including;
   an imaging device configured to image the cell component of the cell or the entire cell contained in the cell container; and
   a control device configured to control the conveyance device and the suction unit based on an imaging result from the imaging device, so that the cell component of the cell or the entire cell is sucked into and held in the chip, in a state in which the chip is connected to the chip connection shaft.
[16] The cell suction system according to any one of [1] to [15], including the chip,
   wherein
   the chip includes:
      the chip base in the shape of a tube forming a connection port on a chip proximal end side; and
      the needle portion in the shape of a tapered tube extending from the chip base to a chip tip end side, and
   the chip connection shaft is connected to the chip base by being inserted and fitted into the chip base via the connection port.

According to the present disclosure, it is possible to provide a cell suction system that can accurately move a needle tip of a needle portion of a chip to the position of a cell to be sucked, after a chip connection shaft of a suction unit is connected to a chip base of the chip held in a chip container.

### BRIEF DESCRIPTION OF THE DRAWINGS

In the accompanying drawings:
FIG. 1 is a schematic diagram illustrating the whole of a cell suction system according to a comparative example of the present disclosure;
FIG. 2 is a schematic diagram illustrating, in the cell suction system illustrated in FIG. 1, a state immediately before a chip connection shaft of a suction unit is connected to a chip base of a chip held in a chip container;
FIG. 3 is a schematic diagram illustrating a state in which the chip connection shaft of the suction unit is connected to the chip base, from the state illustrated in FIG. 2;
FIG. 4 is a schematic diagram illustrating, in a cell suction system according to a first embodiment of the present disclosure, a state in which a chip connection shaft of a suction unit is connected to a chip base of a chip held in a chip container;
FIG. 5A is a detailed diagram illustrating chips, the chip container, and a chip container holder illustrated in FIG. 4, from obliquely above;
FIG. 5B is a detailed diagram illustrating the chips, the chip container, and the chip container holder illustrated in FIG. 5A, from above;
FIG. 5C is a cross-sectional view along A-A of FIG. 5B;
FIG. 6 is a schematic diagram illustrating, in a cell suction system according to a second embodiment of the present disclosure, a state in which a chip connection shaft of a suction unit is connected to a chip base of a chip held in a chip container;
FIG. 7A is a detailed diagram illustrating chips, the chip container, and a chip container holder illustrated in FIG. 6, from obliquely above;
FIG. 7 B is a detailed diagram illustrating the chips, the chip container, and the chip container holder illustrated in FIG. 7A, from above;
FIG. 7C is a cross-sectional view along B-B of FIG. 7B;
FIG. 7 D is a partly enlarged view of FIG. 7C.

### DETAILED DESCRIPTION

Hereinafter, embodiments of the present disclosure will be exemplarily described in detail with reference to the drawings.

The embodiments relate to cell suction systems 10 that suck a cell component of a cell or an entire cell, and more specifically relate to cell suction systems 10 that are suitable for suction targeting a large number of cells.

In research on life systems, it is common to identify characteristic cells from among many cells in cell culture wells and perform suction operation on those cells or components of those cells. For example, in drug discovery process of discovering or designing new drugs, a drug discovery screening step is conducted to find compounds that indicate efficacy and activity from among many candidate compounds. In this step, cells indicating significantly unique changes are selected from cells in cell culture wells to which the candidate compounds have been given, and those cells or cell components are sucked for analysis such as mass spectrometry.

To suck a cell, cell suction operation into a chip is performed using a suction pipette or a dispensing device equipped with the chip, while the cell to be sucked is confirmed with a microscope. To suck a cell component, cell component suction operation is performed using a fine chip called nanospray chip, while a target cell is confirmed with a microscope.

Aside from the operation of sucking and analyzing specific cells or cell components in cell culture wells, cell analysis systems that capture microscopic images of a large number of cells in the cell culture wells, identify the individual cells by image processing, and calculate the characteristic amounts, such as size and brightness, of each cell in real-time have been put into practical use.

The cell analysis systems can detect characteristic cells and observe temporal changes, based on the calculated characteristic amounts. The characteristic amounts of each cell can be represented in histograms or scatter plots, or displayed in lists. However, the cell analysis systems are limited to analysis based on the characteristic amounts obtained from the images, and detailed analysis of the specific cells, which are identified through this analysis, is not anticipated.

The operation of sucking cells or cell components involves selecting and sucking the cells to be analyzed while visually inspecting the individual cells, which is very labor-intensive and burdensome for an operator when a large number of cells are processed. Therefore, the development of a system that can easily detect candidate cells for analysis from a large number of cells and perform suction operation immediately is desired. For example, the technology disclosed in PTL 1 makes it possible to support suction operation targeting characteristic cells among a large number of cells.

The cell suction systems 10 according to the embodiments relate to cell suction systems 10 that are suitable for suction operation targeting characteristic cells among a large number of cells, and are designed to accurately move a needle tip of a needle portion 332 of a chip 330 to the position of a cell to be sucked, after a chip connection shaft 322 of a suction unit 320 is connected to a chip base 331 of the chip 330 held in a chip container 341.

Before describing the cell suction systems 10 according to the embodiments, a cell suction system 10 in which it is difficult to accurately move a needle tip of a needle portion 332 of a chip 330 to the position of a cell to be sucked, after a chip connection shaft 322 of a suction unit 320 is connected to a chip base 331 of the chip 330 held in a chip container 341 will be described as a comparative example.

The cell suction system 10 according to the comparative example has the same configuration as the cell suction systems 10 according to the embodiments, except that the configuration of a chip container holder 342 is different.

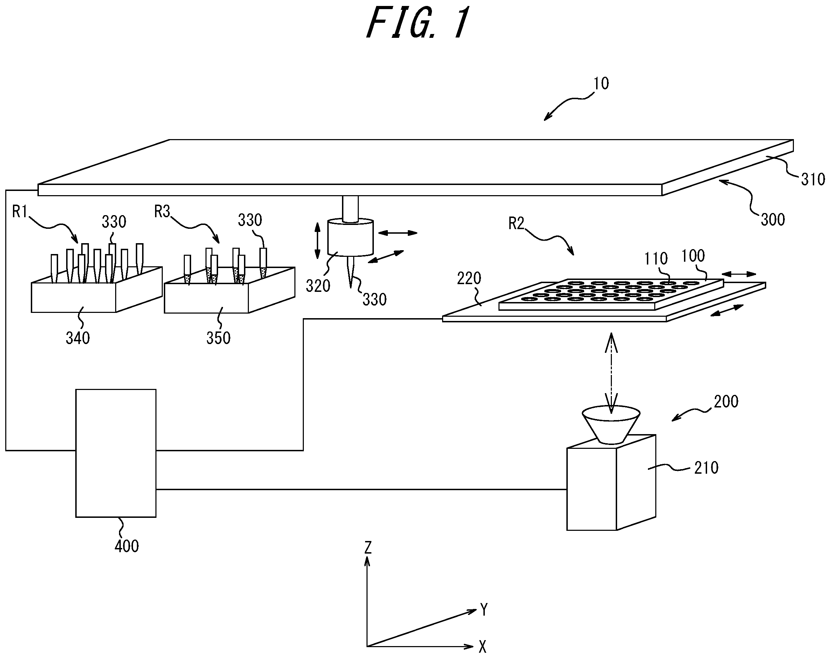

As illustrated in FIGS. 1 to 2, the cell suction system 10 according to the comparative example includes a cell container 100, an imaging device 200, a cell suction device 300, and a control device 400.

The cell container 100 has multiple compartments 110 arranged on an XY plane (preferably a horizontal plane), and each compartment 110 contains cells. The cell container 100 is a cell culture container having cell culture well s as the compartments 110. The cell container 100 is disposed in a cell suction operation area R2.

The imaging device 200 includes an optical system device 210 and an XY stage 220. The optical system device 210 images cell components of cells or entire cells contained in the cell container 100 placed on the XY stage 220, and generates images. The images may be obtained through confocal two-color fluorescence observation, confocal single-color fluorescence observation, epifluorescence single-color observation, bright-field observation, or the like, and are not particularly limited. The XY stage 220 moves the cell container 100 in X and Y directions relative to the optical system device 210 to a position required for observing a desired cell by the optical system device 210.

The cell suction device 300 includes a conveyance device 310 (XYZ stage), a suction unit 320, chips 330, a chip container portion 340, and a post-suction chip container portion 350. The chip container portion 340 is disposed in a chip connection operation area R1. The post-suction chip container portion 350 is disposed in a chip detachment operation area R3.

The conveyance device 310 conveys the suction unit 320 to a desired position in X, Y, and Z directions. The conveyance device 310 conveys the suction unit 320 to a position in which a chip connection shaft 322 is connected to a chip 330 held in a chip container 341 (in the chip connection operation area R1), and after the connection to the chip 330, conveys the suction unit 320 from that position to a position in which a cell component of a cell or an entire cell contained in the cell container 100 is sucked into the chip 330 (in the cell suction operation area R2), and after the suction operation, conveys the suction unit 320 from that position to a position in which the chip 330 is detached and held in a post-suction chip container (in the chip detachment operation area R3).

The suction unit 320 includes a suction unit base 321, the chip connection shaft 322, and a chip disconnection shaft 323. The suction unit base 321 is conveyed by the conveyance device 310. The chip connection shaft 322 is in the shape of a shaft (preferably cylindrical shaft) centered on a first central axis O1 extending downward from the suction unit base 321, and is connected to a chip base 331 of the chip 330. In a state in which the chip 330 is connected to the chip connection shaft 322, the suction unit 320 causes the chip 330 to suck and hold the cell component of the cell or the entire cell from a needle tip of a needle portion 332.

This suction operation may be performed by changing pressure inside the needle portion 332 by the suction unit base 321 through the chip base 331 and a hole (not illustrated) provided in the chip connection shaft 322. The suction operation may also be performed by surface tension generated inside due to the thin tubular tip of the needle portion 332. When suction force due to the surface tension is too strong and not only the desired cell component or entire cell but also surrounding components are sucked, the suction of the surrounding components may be suppressed by applying positive pressure to the needle tip by the suction unit base 321.

The suction operation may be performed by generating an interaction between the needle tip and the cell, such as by changing the height of the needle tip to pierce the cell, and switching pressure to be supplied from the suction unit base 321 to the needle tip between negative pressure and positive pressure according to the timing of the interaction, to suck the desired cell component or entire cell from a desired site. The pressure (negative pressure or positive pressure) to be supplied to the needle tip is provided from a pressure source such as a not-illustrated pump. A pressure supply path from the pressure source to a tip of the chip connection shaft 322 may have a pressure control part such as a valve to control the pressure.

The chip disconnection shaft 323 is held movably up and down relative to the suction unit base 321. In a state in which the chip 330 is connected to the chip connection shaft 322, the chip disconnection shaft 323 moves downward relative to the suction unit base 321 to press down the chip base 331 and disconnect the chip 330, thus detaching the chip 330 from the chip connection shaft 322.

The chip 330 has the chip base 331 in the shape of a tube (preferably cylinder) centered on a second central axis O2, which forms a connection port 331a on a chip proximal end side, and the needle portion 332 in the shape of a tapered tube (preferably tapered cylinder) extending from the chip base 331 to a chip tip end side. The chip connection shaft 322 is connected to the chip base 331 by being inserted and fitted into the chip base 331 via the connection port 331a. In this comparative example, the chip base 331 has a larger outer diameter than the needle portion 332. The chip 330 may also be configured so that the chip base 331 has an outer diameter equal to or less than the needle portion 332. In that case, the configuration of the chip container 341, which holds chips 330, can be set as appropriate according to the shape of the chip 330.

The chip base 331 of the chip 330 is made of an elastic material such as elastomer or resin. By making the chip base 331 of the elastic material, when the chip connection shaft 322 is inserted downward and fitted into the connection port 331a of the chip base 331, the tubular chip base 331 deforms to expand in a radial direction and generates contact pressure on the chip connection shaft 322, which allows the chip base 331 to be connected to the chip connection shaft 322 by frictional force generated by the contact pressure.

A method of forming the needle portion 332 of the chip 330 is not particularly limited. For example, the needle portion 332 can be formed by heating, melting, and pulling a glass tube to create a constriction in the glass tube, and separating the glass tube at the constriction to make the constriction as a sharp needle tip. The needle portion 332 of the chip 330 may also be formed by etching a crystalline material such as silicon with a chemical solution, creating a sharp part due to a difference in etching speed according to the crystal orientation, and making the sharp part as a sharp needle tip.

The chip container portion 340 includes the chip container 341 and a chip container holder 342. The chip container 341 holds multiple chips 330, which are before used for cell suction operation. The chip container 341 has chip base holders 341a each of which holds the chip base 331. The chip base holder 341a is in the shape of a through hole, through which the needle portion 332 passes downward, on which the chip base 331 is placed, and into which an outer peripheral surface of the chip base 331 is fitted. The chip container 341 is not limited to the configuration with the chip base holders 341a, and for example, may be configured to hold the needle portions 332, instead of or in addition to the chip bases 331.

The post-suction chip container portion 350 includes a post-suction chip container (not illustrated) and a post-suction chip container holder (not illustrated). The post-suction chip container holds multiple chips 330, which are after used for cell suction operation. The post-suction chip container is configured in the same manner as the chip container 341, but is not limited to this.

The control device 400 includes a computer that controls the operations of the XY stage 220, the optical system device 210, the conveyance device 310, and the suction unit 320, and controls the conveyance device 310 and the suction unit 320 based on imaging results (images obtained by observing cells) from the imaging device 200, so that the cell component of the cell or the entire cell is sucked into and held in the chip 330, in a state in which the chip 330 is connected to the chip connection shaft 322. The control device 400 displays the imaging results on a display or the like, receives instructions from an operator who is looking at the displayed imaging results, and operates the conveyance device 310 and the suction unit 320 based on the received instructions. The cell suction system 10 is not limited to the configuration with the conveyance device 310 that conveys the suction unit 320. For example, the cell suction system 10 may be configured with a conveyance device, instead of the conveyance device 310, that conveys each of the chip container portion 340, the post-suction chip container portion 350, and the XY stage 220. In this case, the control device 400 may be configured to control the above-described conveyance device and the suction unit 320 so that the cell component of the cell or the entire cell is sucked into and held in the chip 330 based on the imaging results (images obtained by observing cells) from the imaging device 200, in a state in which the chip 330 is connected to the chip connection shaft 322.

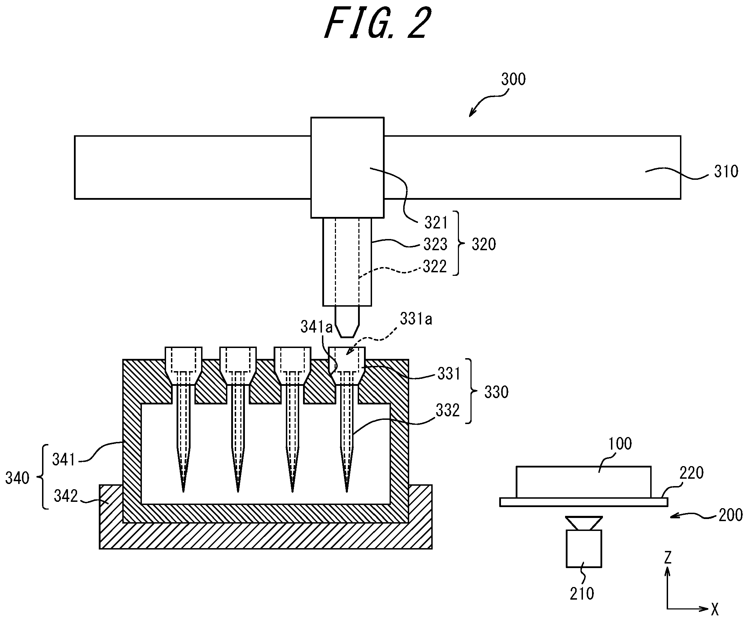

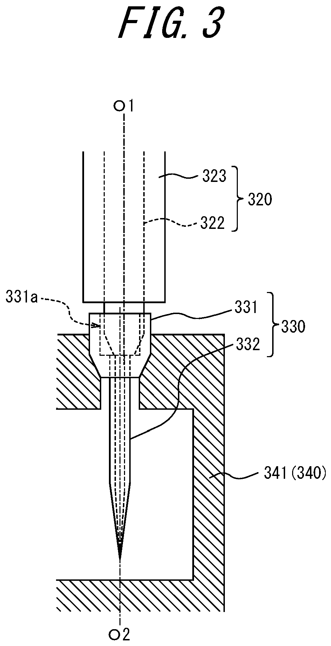

In this comparative example, the chip container holder 342 holds the chip container 341 so that the chip container 341 does not move in the XY plane relative to a base (not illustrated) of the cell suction system 10, which supports the conveyance device 310. Therefore, in this comparative example, as illustrated in FIGS. 2 to 3, in the chip connection operation area R1, when the conveyance device 310 moves the suction unit 320 down and the suction unit 320 is inserted into the connection port 331a of the chip base 331 of the chip 330 held in the chip container 341, axial misalignment, that is, the first central axis O1 of the chip connection shaft 322 is misaligned with the second central axis O2 of the chip base 331 may occur. In such a case, it becomes difficult to accurately move the needle tip of the needle portion 332 of the chip 330 to the position of the cell to be sucked.

That is, as illustrated in FIGS. 2 to 3, when the chip connection shaft 322 descends at a position that is axially misaligned with the chip base 331 in the X or Y direction and is inserted into the chip base 331, the chip base 331 cannot move on the XY plane because the chip container 341 is installed in the base of the cell suction system 10 so as not to move on the XY plane. This results in the insertion into the chip base 331 in a state of being deformed in a direction axially misaligned with the chip connection shaft 322. As a result, residual stress biased in a circumferential direction occurs in the chip base 331.

Therefore, when the conveyance device 310 subsequently moves the chip 330 up from the chip container 341 by ascending the suction unit 320 to move the chip 330 to the cell suction operation area R2 in which suction operation is performed, the residual stress in the chip base 331 is gradually released. Accordingly, the needle tip of the needle portion 332 moves on the XY plane, which results in variations in the position of the needle tip.

The cell suction operation area R2 may be heated to a temperature such as 37°C to prevent cell death. In such a case, the ambient temperature of the chip connection operation area R1 is usually lower than that of the cell suction operation area R2, so the conveyance to the cell suction operation area R2 is more susceptible to the effects of the residual stress.

In particular, when a component of a specific area in the cell is sucked, it is necessary to align the needle tip on the XY plane with an accuracy of a few micrometers (for example, 2 µm) or less. However, it is difficult to control the position of the chip connection shaft 322 of the suction unit 320 relative to the chip 330 held in the chip container 341 by operation teaching of the conveyance device 310, so that the misalignment of the needle tip due to the residual stress remains within the above-described accuracy range, which leads to problems such as requiring a special jig or tool for teaching or taking a significant amount of time for teaching.

Additionally, since the chip container 341 is often produced by resin molding, there are variations in the shape of the chip container 341 itself. This leads to variations in the position of the chip 330 held in the chip container 341.

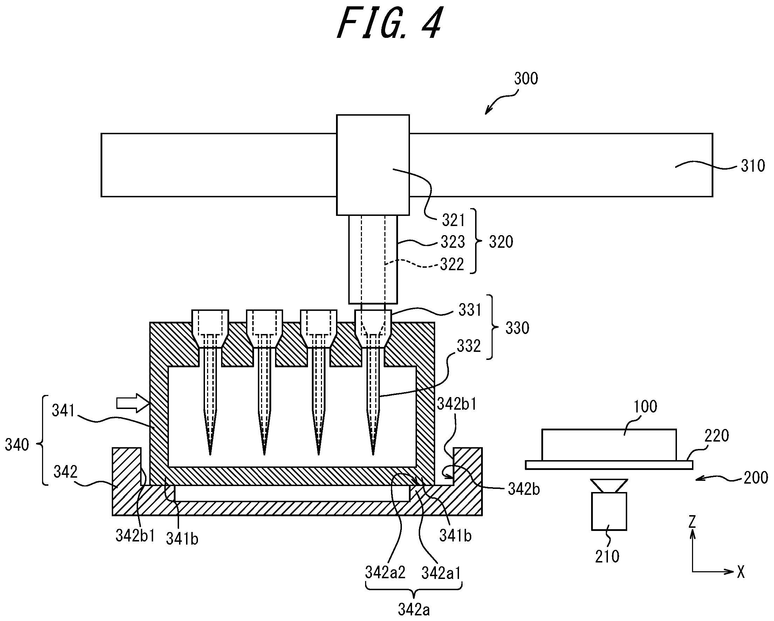

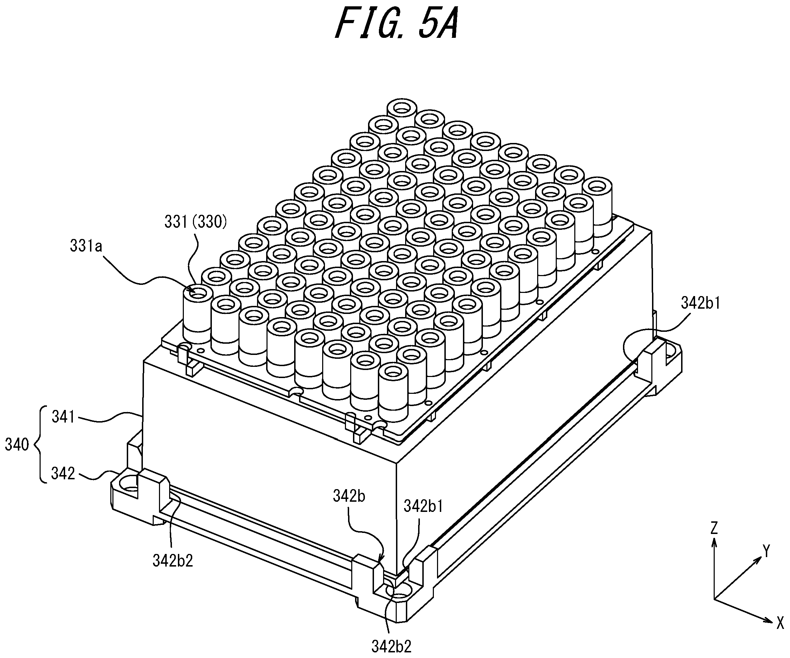

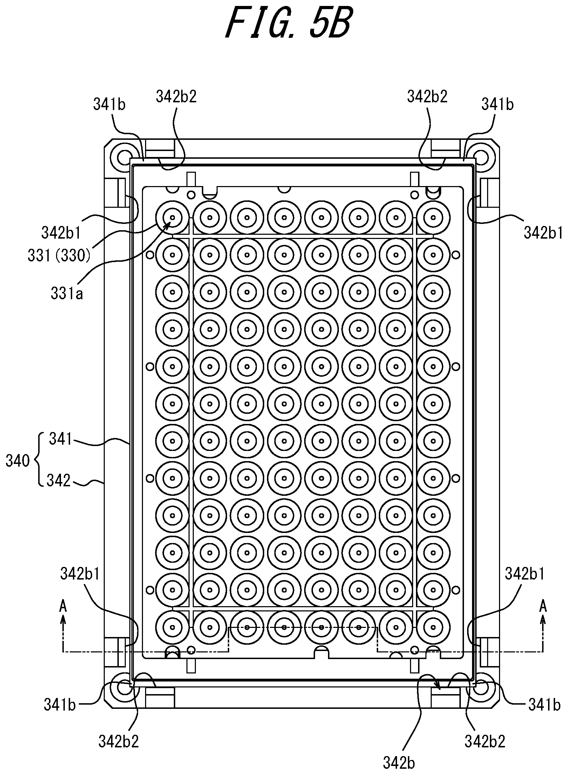

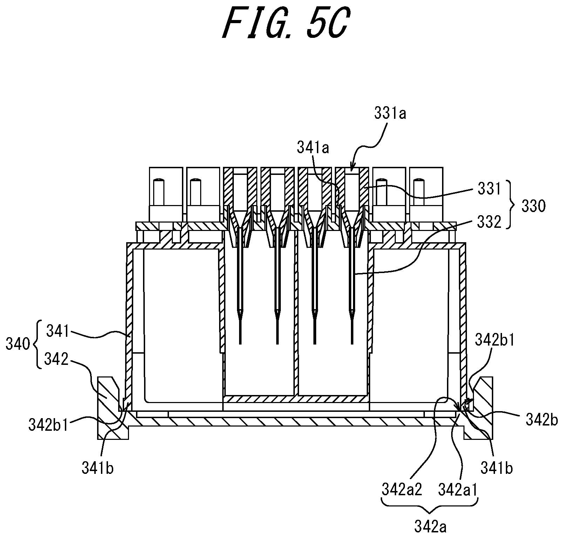

In order to address the problem of the misalignment of the needle tip due to the axial misalignment during connection in this comparative example, in a cell suction system 10 according to a first embodiment of this disclosure, as illustrated in FIGS. 4 to 5C, a chip container holder 342 holds a chip container 341 so as to allow movement of chips 330 and the chip container 341 relative to a chip connection shaft 322, thereby eliminating the axial misalignment when the chip connection shaft 322 is connected to a chip base 331 of a chip 330 held in the chip container 341.

The chip container holder 342 has a placement portion 342a on which the chip container 341 is placed. The chip container 341 has four corners 341b in a top view, and in this embodiment, only the four corners 341b of the chip container 341 are placed on the placement portion 342a of the chip container holder 342 to reduce sliding resistance. The placement portion 342a is in the shape of a plane along an XY plane. The placement portion 342a is preferably as slippery as possible. For this reason, the placement portion 342a is preferably configured with a base material 342a1 and a surface layer 342a2, which is applied to an upper surface of the base material 342a1 and has a lower coefficient of friction than the upper surface. The surface layer 342a2 is formed by PTFE coating, PTFE composite electroless nickel plating, or the like. Alternatively, the entire chip container holder 342 may be made of a material with a low coefficient of friction such as PTFE.

In this embodiment, the chip container holder 342 has a chip container regulator 342b, which regulates movement of the chip container 341 in a horizontal direction while allowing smooth sliding of the chip container 341 on the placement portion 342a, to eliminate the axial misalignment when the chip connection shaft 322 is connected to the chip base 331 of the chip 330 held in the chip container 341. The chip container regulator 342b has four X-directional opposite surfaces 342b1 and four Y-directional opposite surfaces 342b2, which are opposite each other with leaving gaps in the X and Y directions at each of the four corners 341b of the chip container 341. The chip container regulator 342b is constituted of four upward protrusions with the four X-directional opposite surfaces 342b1 and four upward protrusions with the four Y-directional opposite surfaces 342b2, but is not limited to this. The chip container regulator 342b allows movement of the chip container 341 on the XY plane (movement in the X direction, movement in the Y direction, and rotation associated with these movements).

This embodiment can address the problem of the misalignment of the needle tip due to the axial misalignment during connection as described above, because while the chips 330 and the chip container 341 are held with movement on the XY plane being regulated by the chip container regulator 342b, movement of the chips 330 and the chip container 341 to eliminate the above axial misalignment can be allowed within the range of that regulation by smooth sliding on the placement portion 342a.

The chip container holder 342 may eliminate the axial misalignment by connecting the chip connection shaft 322 to the chip base 331 of the chip 330 held in the chip container 341, and then performing disconnection and connection one or more times. However, it is preferable from the viewpoint of operation efficiency to have a configuration that eliminates the misalignment with connection of a single time.

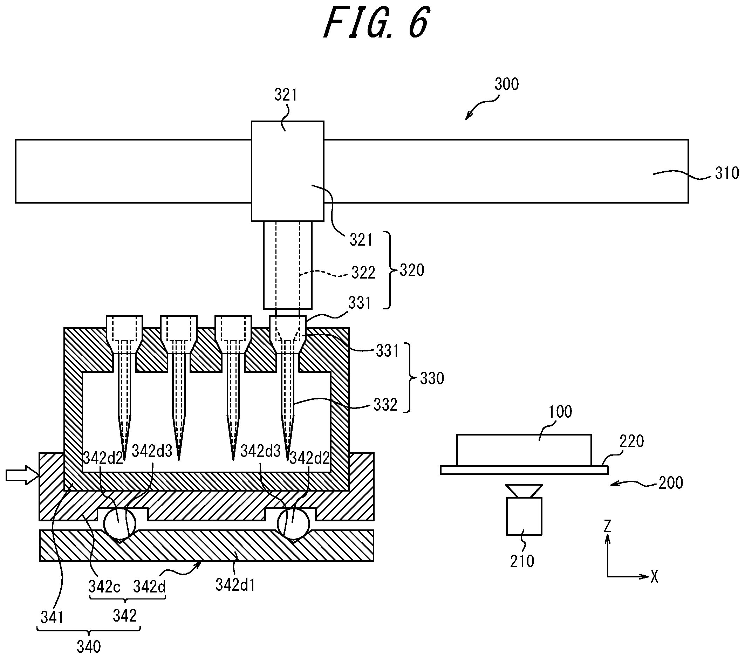

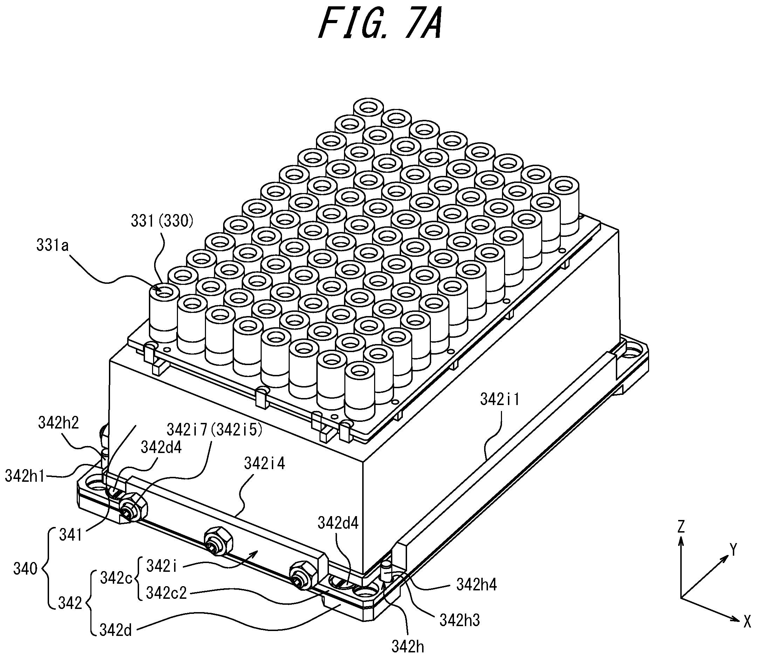

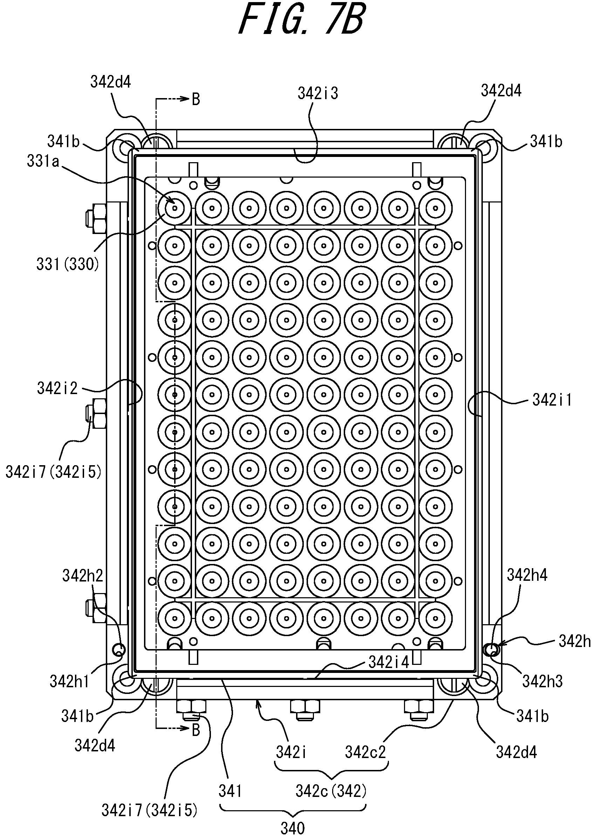

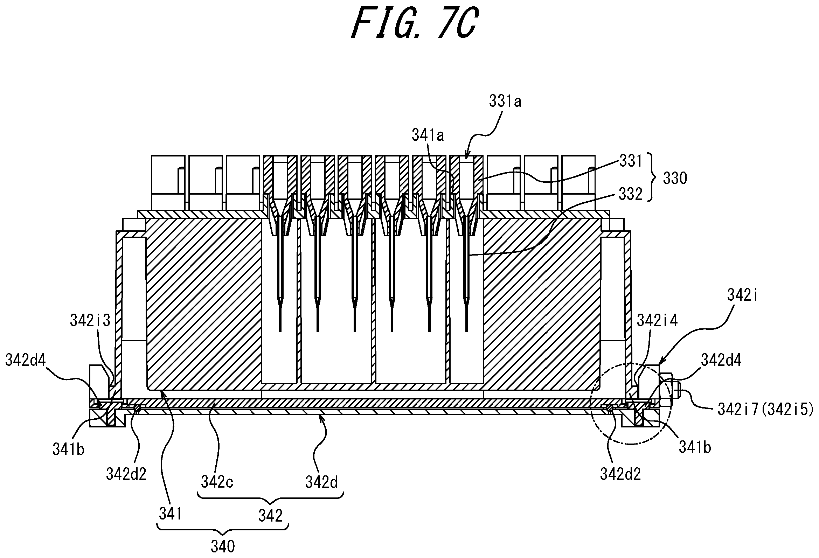

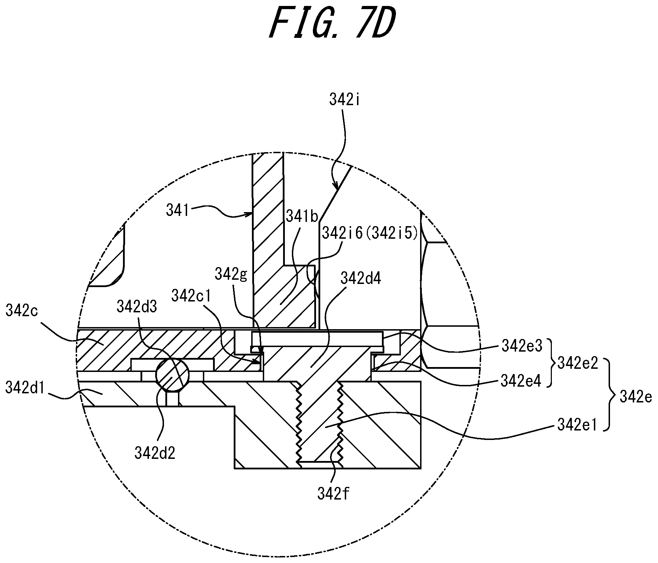

As in a second embodiment illustrated in FIGS. 6 to 7D, the chip container holder 342 may be configured with a first holder 342c that holds the chip container 341, and a second holder 342d that holds the first holder 342c while allowing movement of the first holder 342c relative to the second holder 342d in the horizontal direction to eliminate the axial misalignment when the chip connection shaft 322 is connected to the chip base 331 of the chip 330 held in the chip container 341.

The second holder 342d has a second holder main body 342d1 and three or more ball-shaped rolling elements 342d2, which are rollably held by the second holder main body 342d1 and on which the first holder 342c is placed. As illustrated, one rolling element 342d2 is preferably provided in the vicinity of each of the four corners 341b of the chip container 341. The rolling element 342d2 is preferably also provided in the vicinity of the center of the chip container 341 in a bottom view.

The second holder main body 342d1 has reduced diameter support surfaces 342d3, whose diameter gradually reduces downward and which rotatably support the rolling elements 342d2 by slidably abutting lower surfaces of the rolling elements 342d2, and an ascent regulation portion 342d4, which regulates upward movement of the first holder 342c relative to the second holder main body 342d1 so that the rolling elements 342d2 do not get out of the reduced diameter support surfaces 342d3. The ascent regulation portion 342d4 regulates the ascent of the first holder 342c relative to the second holder main body 342d1, from a state in which the first holder 342c is placed on the rolling elements 342d2, by an ascent width that is required of the rolling elements 342d2 to get out of the reduced diameter support surfaces 342d3 or more. Therefore, the rolling elements 342d2 are favorably held in the reduced diameter support surfaces 342d3.

The ascent regulation portion 342d4 is configured with one or more (in this embodiment, multiple) securing screws 342e. The securing screw 342e has a screw shaft 342e1 attached to a female screw 342f provided in the second holder main body 342d1, and a screw head 342e2 connected to an upper end of the screw shaft 342e1. The screw head 342e2 has a large diameter portion 342e3. A small diameter portion 342e4, which has a smaller outer diameter than the large diameter portion 342e3, is provided between the large diameter portion 342e3 and the screw shaft 342e1. The first holder 342c has an opening 342c1 the size of which passes the screw shaft 342e1 and the small diameter portion 342e4, but does not pass the large diameter portion 342e3. Therefore, the ascent regulation portion 342d4 regulates the ascent of the first holder 342c relative to the second holder main body 342d1, by the first holder 342c abutting a lower surface of the large diameter portion 342e3 of the securing screw 342e at the periphery of the opening 342c1. In this embodiment, the amount of screwing the screw shaft 342e1, which determines an allowable ascent width, is determined by abutting a lower surface of the small diameter portion 342e4 on an upper surface of the second holder main body 342d1, but is not limited to this. For example, an annular collar member with an inner diameter larger than the outer diameter of the screw shaft 342e1 may be separately provided between the large diameter portion 342e3 and the upper surface of the second holder main body 342d1, and the amount of screwing may be determined by the width of the collar member in a vertical direction.

Additionally, a gap 342g is provided between the small diameter portion 342e4 of the securing screw 342e and the outer peripheral edge of the opening 342c1, to allow movement (X-directional movement, Y-directional movement, and rotation accompanying these movements) of the chips 330, the chip container 341, and the first holder 342c relative to the chip connection shaft 322, to eliminate the axial misalignment described above. Therefore, this embodiment can address the problem of the misalignment of the needle tip due to the axial misalignment, because while the chips 330, the chip container 341, and the first holder 342c are held with movement on the XY plane being regulated by the small diameter portions 342e4 of the securing screws 342e, movement of the chips 330, the chip container 341, and the first holder 342c to eliminate the above axial misalignment can be allowed within the range of that regulation.

In this embodiment, the chip container holder 342 has a positioning portion 342h, which changes the state between a positioning state in which the first holder 342c is positioned on the XY plane relative to the second holder 342d and a positioning release state in which the positioning is released. The positioning portion 342h preferably performs positioning at a location that allows movement to eliminate the axial misalignment in all directions on the XY plane, and more preferably positioning at the center of the range of regulation with which movement on the XY plane is regulated. According to the positioning portion 342h, when the position of the chip connection shaft 322 of the suction unit 320 is set relative to the chip 330 held in the chip container 341 by operation teaching of the conveyance device 310, the chip container holder 342 can be kept in an appropriate positioning state that can favorably address the problem of the misalignment of the needle tip due to the axial misalignment.

The positioning portion 342h is configured with a circular first through hole 342h1, in a top view, provided in the first holder 342c, a circular first hole (not illustrated), in a top view, provided in the second holder 342d, a first pin 342h2 insertable into and removable from the first through hole 342h1 and the first hole, an elongated circular second through hole 342h3, in a top view, provided in the first holder 342c, a circular second hole (not illustrated), in a top view, provided in the second holder 342d, and a second pin 342h4 insertable into and removable from the second through hole 342h3 and the second hole. According to the above configuration, the chip container holder 342 can be set to the positioning state by inserting the first pin 342h2 into the first through hole 342h1 and the first hole and inserting the second pin 342h4 into the second through hole 342h3 and the second hole, and can be set to the positioning release state by removing the first pin 342h2 and the second pin 342h4. Since the second through hole 342h3 has an elongated circular shape in a top view, it is possible to absorb manufacturing errors and the like of the first holder 342c and the second holder 342d.

The first holder 342c includes a first holder main body 342c2 on which the chip container 341 is placed, and a chip container positioning portion 342i, which positions the chip container 341 on the first holder main body 342c2. The chip container positioning portion 342i is configured with an X-directional pressing device 342i2 that presses the chip container 341 against an X-directional receiving surface 342i1, which is provided in the first holder main body 342c2 and receives the chip container 341 in the X direction, a Y-directional pressing device 342i4 that presses the chip container 341 against a Y-directional receiving surface 342i3, which is provided in the first holder main body 342c2 and receives the chip container 341 in the Y direction. The chip container positioning portion 342i can more favorably address the problem of the misalignment of the needle tip due to the axial misalignment.

Each of the X-directional pressing device 342i2 and the Y-directional pressing device 342i4 is configured with, for example, multiple (three in this embodiment) ball plungers 342i5 each of which is constituted of a ball 342i6 that contacts the chip container 341, a ball housing 342i7 that houses the ball 342i6 in an advanceable and retreatable manner, and an elastic body (not illustrated) such as a compression spring that presses the ball 342i6 against the chip container 341 in a forward direction. The ball plungers 342i5 allow smooth placement of the chip container 341 onto the first holder 342c. However, each of the X-directional pressing device 342i2 and the Y-directional pressing device 342i4 may be configured to perform pressing by deformation of elastic bodies such as leaf springs or rubber, without using the ball plungers 342i5.

The embodiments of the present disclosure have been described above, but the present disclosure is not limited to the embodiments described above. The above embodiments can be variously modified within the scope of not departing from the gist of the present disclosure.

## Claims

1. A cell suction system comprising:
a suction unit including a chip connection shaft to be connected to a chip base of a chip held in a chip container, the suction unit causing the chip to suck and hold a cell component of a cell or an entire cell from a needle tip of a needle portion, in a state in which the chip is connected to the chip connection shaft; and
a chip container holder that holds the chip container so as to allow movement of the chip and the chip container relative to the chip connection shaft, to eliminate axial misalignment when the chip connection shaft is connected to the chip base of the chip held in the chip container.

2. The cell suction system according to claim 1, comprising the chip container,
wherein the chip container holder includes:
a placement portion on which the chip container is placed; and
a chip container regulator that regulates movement of the chip container in a horizontal direction while allowing sliding of the chip container on the placement portion, to eliminate the axial misalignment when the chip connection shaft is connected to the chip base of the chip held in the chip container.

3. The cell suction system according to claim 2, wherein
the chip container has four corners in a top view, and
only the four corners of the chip container are placed on the placement portion of the chip container holder.

4. The cell suction system according to claim 1, wherein
the chip container holder has a placement portion on which the chip container is placed, and
the placement portion includes:
a base material; and
a surface layer applied to an upper surface of the base material, the surface layer having a lower coefficient of friction than the upper surface.

5. The cell suction system according to claim 1, comprising:
a first holder that holds the chip container; and
a second holder that holds the first holder while allowing movement of the first holder relative to the second holder, to eliminate the axial misalignment when the chip connection shaft is connected to the chip base of the chip held in the chip container.

6. The cell suction system according to claim 5, wherein the second holder includes:
a second holder main body; and
a rolling element rollably held by the second holder main body, the first holder being placed on the rolling element.

7. The cell suction system according to claim 6, wherein
the rolling element is in shape of a ball, and
three or more rolling elements are provided.

8. The cell suction system according to claim 6, wherein the second holder main body includes:
a reduced diameter support surface whose diameter gradually reduces downward, the reduced diameter support surface rotatably supporting the rolling element by slidably abutting a lower surface of the rolling element; and
an ascent regulation portion that regulates upward movement of the first holder relative to the second holder main body, so that the rolling element does not get out of the reduced diameter support surface.

9. The cell suction system according to claim 5, wherein the chip container holder includes a positioning portion that changes a state between a positioning state in which the first holder is positioned relative to the second holder in a horizontal direction and a positioning release state in which positioning is released.

10. The cell suction system according to claim 5, wherein the first holder includes:
a first holder main body on which the chip container is placed; and
a chip container positioning portion that positions the chip container on the first holder main body.

11. The cell suction system according to claim 1, comprising:
a chip connection operation area in which the chip connection shaft is connected to the chip held in the chip container; and
a cell suction operation area in which the cell component of the cell or the entire cell is sucked into the chip,
wherein an ambient temperature of the chip connection operation area is lower than that of the cell suction operation area.

12. The cell suction system according to claim 1, comprising:
an imaging device configured to image the cell component of the cell or the entire cell contained in a cell container; and
a control device configured to control the suction unit based on an imaging result from the imaging device, so that the cell component of the cell or the entire cell is sucked into and held in the chip, in a state in which the chip is connected to the chip connection shaft.

13. The cell suction system according to claim 1, comprising a conveyance device configured to convey the suction unit from a position in which the chip connection shaft is connected to the chip held in the chip container, to a position in which the cell component of the cell or the entire cell contained in a cell container is sucked into the chip.

14. The cell suction system according to claim 13, comprising;
an imaging device configured to image the cell component of the cell or the entire cell contained in the cell container; and
a control device configured to control the conveyance device and the suction unit based on an imaging result from the imaging device, so that the cell component of the cell or the entire cell is sucked into and held in the chip, in a state in which the chip is connected to the chip connection shaft.

15. The cell suction system according to claim 1, comprising the chip,
wherein
the chip includes:
the chip base in shape of a tube forming a connection port on a chip proximal end side; and
the needle portion in shape of a tapered tube extending from the chip base to a chip tip end side, and
the chip connection shaft is connected to the chip base by being inserted and fitted into the chip base via the connection port.
